# EUROPEAN PATENT APPLICATION

(11) **EP 2 199 800 A1**
(43) Date of publication of application: **23.06.2010**
(21) Application number: 09179155.8
(22) Date of filing: 14.12.2009
(51) Int. Cl.: G01N 33/68, H01J 49/00

(54) **A method of more reliable protein determination from mass spectrometric data**

(30) Priority: 15.12.2008 CZ 20080803
(71) Applicant: Microbiology Institute AS CR, v.v.i., 142 20 Praha 4 (CZ)
(72) Inventor: Strohalm, Martin, 160 00 Praha 6 (CZ); Novak, Petr, 700 30 Ostrava (CZ); Havlicek, Vladimir, 142 00 Praha 4 (CZ); Pompach, Petr, 250 63 Mratin (CZ); Man, Petr, 252 42 Vestec (CZ); Kavan, Daniel, 560 02 Ceska Trebova (CZ)
(74) Representative: Beetz & Partner

(57) **Abstract**

Compared to current methods, using the novel approach for protein analysis, up to 7-fold increase of identified peptides can be reached. This improves better sequence coverage and enables identification of post-translational modifications or important changes in amino acids, peptides, proteins. More reliable protein quantitation results are obtained.

## Description

### Technology field

Protein identification and quantitation by mass spectrometry

### Background of the art

Nowadays there are many different approaches utilizing mass spectrometry for protein analysis and many different hardware setups of mass spectrometers are available. Mass spectrometry is almost routinely used for qualitative and quantitative analysis of proteins or for combination of both. Proteins are identified based on its building blocks, amino acids constituents that represent the analyzed peptides. Every protein consists of characteristic sequence parts (peptides), and analysis of these peptides is used for protein identification. The more peptides from particular protein are correctly identified, the longer range of the sequence is covered and the identification is more relevant. Current analytical methods use the most abundant peptides only and identification of less abundant proteins is not therefore possible.

There is a variety of protein modifications which are not predictable and may increase the sequence area which cannot be described by standard methods. Determination of such a modification may not only improve the identification but also can reveal some important biological information about a drug impact, cell process, etc.

There is a tight relationship between the protein identification and quantitation. In the cases where particular protein is not identified correctly, the information about its quantitation is not correct as well. Using low-resolution methods, two peptides with similar molecular weights can easily be identified as a single peptide with incorrect concentration. Corresponding protein expression changes are therefore misleading and subsequent analyses and experiments could be pointless.

### Disclosure of the invention

The critical aspect of protein identification is to identify as much of the protein sequence as possible. The sequence is identified in the form of short parts, peptides, with very high mass accuracy (at the least 2 ppm) to eliminate false positive results.

To acquire more relevant data, FTICR-MS system is used for its high mass accuracy. Every peptide detected with sufficient intensity is fragmented for its identification. Measured MS/MS spectra are used to determine the amino acid sequence of a particular peptide. A protein is identified according to its specific peptides which are either in sufficient amounts and or represent other peptides that are for this protein evident and relevant.

Theoretical masses of positively identified peptides of a protein are calculated and used for recalibration of the whole data set to improve mass accuracy of both, identified and *yet unidentified* peptides. This recalibration improves the measured data and results reliability.

For such a high mass accuracy of measured components it is possible to estimate compound composition and assign the correct peptide sequence with reasonable probability.

In common analytical methods, only the most abundant peptides are used for subsequent fragmentation and sequence determination (peptide identification). Therefore, this may cause a serious problem in identifying low abundant peptides. After data recalibration, high mass accuracy of measured peptides can be used for identification of unknown (*yet missed*) components.

The unknown components are searched against theoretical peptides which are proposed.

Theoretical peptides are therefore generated based on the characteristics of the peptide mixture and their theoretical molecular weights are calculated and compared to the measured data. These theoretical peptides should be present in the mixtures either intact or with posttranslational modification(s). The use of these hypothetical peptides represents a dominating claim in this invention.

This method can also be used in quantitation experiments, where exact information about protein changes can help to explain the effect of a drug treatment, cell processing/ signaling, etc.

Peptides with post-translational modifications or with other amino acid transformation(s) are identified by *in silico* applying possible modifications to peptides of already identified proteins.

The proposed peptides, characterized by their precise molecular weights, are compared with the actual acquired and re-calibrated data. If the measured values match to the proposed molecular weights, another (different) sequence region of a protein can be assigned to a matching peptide either with a post-translational modification or other transformation (mutation).

For getting a better sequence coverage, the whole measured datasets are compared with those of peptides that contain: the identified peptides of a protein, the non-identified peptides of the same protein, the peptides with a post-translational modification or other transformation (mutation).

Comparing to previous common methods, using this novel approach up to 7-fold increase in identified peptides can be reached. This improves better sequence coverage and enables one the identification of post-translational modifications or important changes in the amino acids, peptides, proteins.

Novel method can be used for exact quantitation of peptides as well. High mass accuracy can eliminate the incorrect assignment of peptides with close molecular weights and the subsequent quantitation error. Figure 1 illustrates the example of two peptides with close molecular weights which cannot be distinguished without high resolution. In addition, protein quantification can be calculated from more peptides. Figure 2 shows poor quantitative results obtained by standard (currently used) approaches. Figure 3 illustrates the quantitative results achieved by common and novel approaches.

### Figure

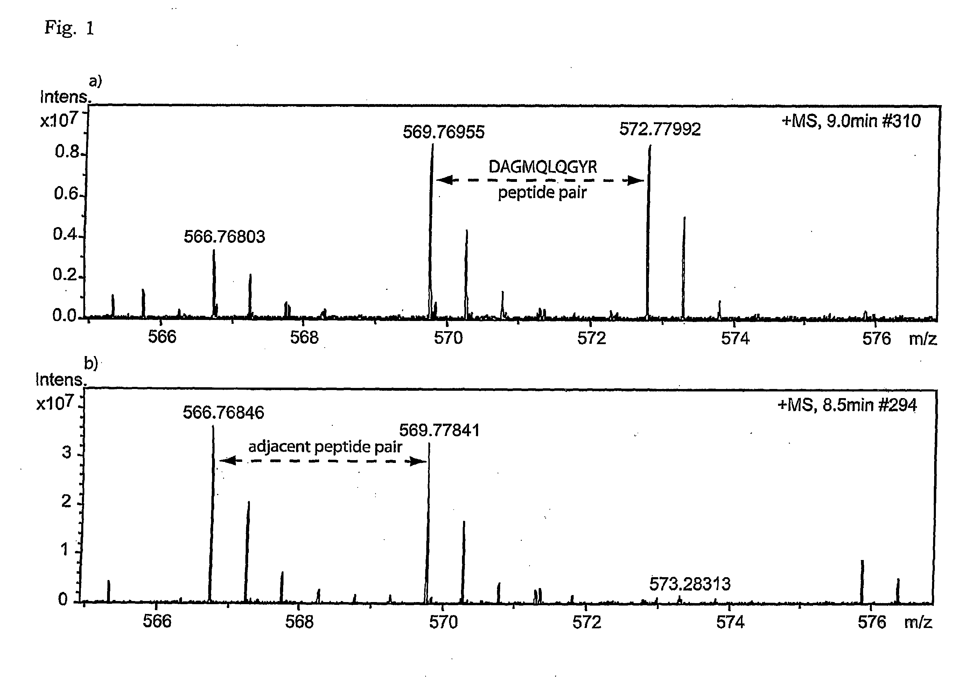

Fig. 1 Mass spectra from a homogenized cell lysate. Accurate masses of two coeluting peptides, a light form of DAGMQLQGYR and heavy form of a completely different peptide are very close (0.009 Da).

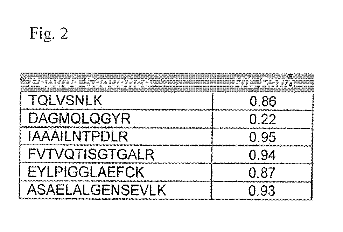

Fig. 2 Table of mutual peptide relative ratios identified in control/monitored sample (H/L) by a standard (common) method for Glutamic-oxaloacetic transaminase 2.

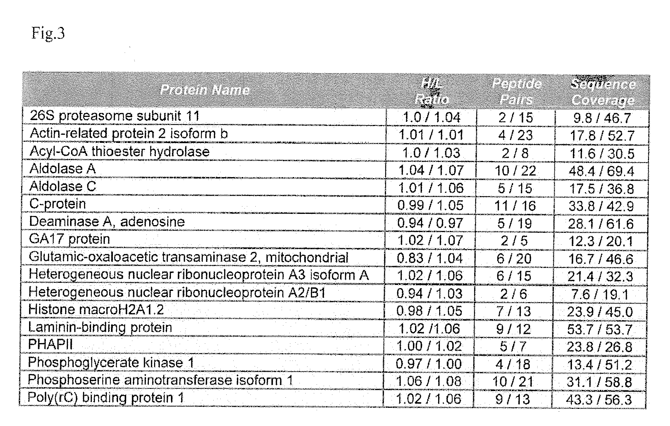

Fig. 3 Results comparison for both (standard and new method) for the analysis of 17 protein species. For all the analyzed proteins, the increase of the number of identified peptides and increased sequence coverage can be clearly seen.

### Examples of the invention

The study of a protein expression changes for acute leukemia cells treated by paclitaxel

The influence of paclitaxel treatment on human acute T-lymphoblastic leukemia cell line CEM was studied by monitoring protein expression changes. Cell cultivation was carried out with concentration of 0.25 x 10⁶ cells/ml in RPMI culture medium supplemented with 20% FSCI, at 37 °C and 5% CO₂, in the presence of 9 x 10⁻⁶ µg/ml of paclitaxel for 120 minutes. Control culture (without paclitaxel) was incubated on the same medium containing isotopically labeled arginine and lysine ¹³C. The experiment was terminated when caspase-9 activity reached 25%. Before the cell lysis, both the control and treated cultures were mixed together in a 1:1 ratio. The lysis was carried out in a buffer containing 20mM Tris-HCl, 6M urea, 100mM dithiothreitol, 1% Triton X-100, 0.5% SDS and 10 units of benzonase endonuclease. 250µl of the mixture of total lysates of labelled and non-labelled cultures was loaded onto a preparative SDS-PAGE with 7-15% gradient gel. After separation, gel tube was sliced to strips of 2 mm in height for in-gel reduction (by 40mM tris(2-carboxyethyl)phosphine, 90 °C, 15 min), alkylation (by 40mM iodoacetamide, room temperature, 90 min) and trypsin digestion (1 µg/fraction). The resulting peptide mixture was separated by liquid chromatography on reversed phase Magic C18AQ column (5µ, 200Å, 0.2 x 150 mm) with a gradient elution ranging from 5-35% of solution B within 40 minutes (A: 0.2% formic acid in 5% MeCN/water and B: 0.2% formic acid in 95% MeCN/water). The eluted peptides were analyzed by FTICR mass spectrometer with ESI ion source and 1.5 ppm mass accuracy. External calibration was done on arginine clusters. Data were analyzed by commercially available software Data Analysis 3.4, BioTools 3.2, WARP-LC 1.1 and in-house Mascot Server. Seventeen different proteins were identified in the 40kDa fraction.

In the case of identified peptide DAGMQLQGYR, where the peptide ratio should be 1:1, measured data suggested 4.5-fold increase of this peptide in the control cell line. This incorrectly indicated some significant changes in gene expression of the corresponding protein caused by poor common algorithms. After reprocessing the data by the novel method presented here, it was found that there are two different peptides in the sample with mass difference of 0.009 Da only, both with 1:1 ratio.

The list of identified peptides was enlarged by missing peptide sequences from the proteins identified by standard search engines (e.g. Mascot, Sequest). This enlarged dataset was *in silico* further enlarged by a complete set of posttranslational modifications. The large virtual peptide database originated this way was then successfully applied to conventional data set and two positive results were obtained. Primarily, the sequence coverage necessary for correct quantitative data was dramatically increased. Secondly, a set of posttranslational modifications that had undergone during the cell treatment was originally determined. Without the reported novel approach, these two new positive outcomes could not be reached.

### Industrial applicability

Protein identification, study of drug influence on gene expression, determination of mutations and post-translational modifications.

## Claims

1. The method of a more reliable protein determination from mass spectrometric data **characterized in that** determination is implemented from at least 2 ppm accurate data; theoretical masses of positively identified peptides of a protein are calculated and used for recalibration of the whole measured data, these recalibrated data are compared with masses of proposed theoretical peptides and non-identified peptides of a protein.

2. The method of a more reliable protein determination from data obtained by mass spectrometry according to claim 1 **characterized in that** the proposed theoretical peptides contain coded amino acid residues.

3. The method of a more reliable protein determination from data obtained by mass spectrometry according to claim 1 or 2 **characterized in that** the proposed theoretical peptides contain coded amino acid residues with posttranslational modification(s).

4. The method of a more reliable protein determination from data obtained by mass spectrometry according claim 1 or 2 or 3 **characterized in that** the proposed theoretical peptides contain non-coded amino acid residues.
